# EUROPEAN PATENT SPECIFICATION

(11) **EP 1 570 809 B1**
(45) Date of publication and mention of the grant of the patent: **14.01.2009**
(21) Application number: 05004289.4
(22) Date of filing: 28.02.2005
(51) Int. Cl.: A61F 2/24

(54) **Cardiac-valve prosthesis**
Herzklappenprothese
Prothèse de valvule cardiaque

(30) Priority: 03.03.2004 IT TO20040135
(43) Date of publication of application: 07.09.2005
(73) Proprietor: SORIN BIOMEDICA CARDIO S.R.L., 13040 Saluggia (Vercelli) (IT)
(72) Inventor: Bergamasco, Giovanni, 10137 Torino (IT); Burriesci, Gaetano, 90135 Palermo (IT); Stacchino, Carla, 10132 Torino (IT)
(74) Representative: Bosotti, Luciano

(56) References cited:
- WO-A-97/24989
- WO-A-03/003943
- US-A1- 2002 138 138
- US-B1- 6 425 916
- US-B1- 6 482 228

## Description

The present invention relates to cardiac-valve prostheses.

The invention has been developed with particular attention paid to cardiac-valve prostheses that can be implanted, for example, via percutaneous route, typically via catheterism as described in the document No. US-A-2002/0042651, or resorting to thoracic-microsurgery techniques. The preferential reference to this context of use must not, however, be interpreted as in any way limiting the scope of the invention.

In the course of the last few years there has begun to be considered to an ever-increasing extent the possibility of using, as an alternative to traditional cardiac-valve prostheses, whether of a mechanical type or of a biological type, valves designed to be implanted via percutaneous route (the so-called "percutaneous valves").

Implantation of a percutaneous valve (or implantation using thoracic-microsurgery techniques) is in fact a far less invasive act than the surgical operation required for implanting a cardiac-valve prosthesis of a traditional type.

Even though it is a relatively recent technology, a rather extensive and articulated literature has already been dedicated to percutaneous valves. In particular, the patent literature on the subject can be viewed as organized according to a certain number of fundamental categories.

A first category of the technique has concentrated on the development of a percutaneous valve prosthesis consisting substantially of a single structure comprising an anchoring element - which is able to support and fix the valve prosthesis stably in the implantation position - and prosthetic valve elements stably connected to the anchoring element - generally in the form of leaflets or flaps, which are able to regulate the blood flow according to criteria aimed at reproducing natural blood flow in the most faithful way possible.

The anchoring element can present a wide range of conformations, presenting, for example, the form of an annular structure consisting of one or more rings (US-A-4 056 854, US-A-5 370 685, US-A-5 545 214, US-A-5 824 064 and WO-A-02 41 789), of a so-called "safety pin" (US-A-4 994 077), of an expandable stent with an open-work or reticular tubular wall (US-A-5 411 552, EP-A-0 850 607, DE-A-19 546 692, US-A-5 840 081, US-A-5 855 597, US-A-5 855 601, US-A-5 957 949, WO-A-00 41652, US-B1-6 168 614, US-A-2001/0 007 956, US-A-2001/0 010 017, WO-A-01 76510, US-B1-6 454 799, US-A-6 482 228, WO-A-03 003943), or again of an expandable stent consisting of a sheet or film wound in an optionally open-worked spiral (US-A-5 925 063, WO-A-02 076348).

Prosthetic-valve elements usually consist of biocompatible material and generally have a structure of a membrane or leaflet type that enables connection thereof to the anchoring element according to various known techniques. There has, however, also been envisaged the use, instead of valve leaflets, of an open/close element in the form of a disk, as in US-A-4 994 077.

A second category of the technique has instead developed percutaneous cardiac-vaive prostheses formed by two separate structures, an anchoring element and a set of prosthetic-valve elements co-ordinating with the anchoring element. The two structures are implantable in succession one after another, with the anchoring element set in position first. An example of such a valve prosthesis with so-called "double structure" is represented by a stent in the form of a cage, with a function of support and anchoring of the valve, to which is associated a valve element in the form of an arrest ball, as described in US-A-5 332 402 and US-A-5 397 351, or of an umbrella, as described in US-A-5 332 402.

Again, the patent literature describes elements of support and/or anchoring for valve prostheses - which can be used with valve prostheses of a different nature - having structures of the stent type, as described in DE-A-19 857 887 and WO-A-01 62189 or of an annular type, as described in US-A-5 980 570, EP-A-1 088 529 and WO-A-01 64137.

Other patent documents concerning percutaneous cardiac-valve prostheses and corresponding methods of implantation are US-A-3 671 979, US-A-4 777 951 and US-A-5 954 766.

More specifically, the invention relates to a cardiac-valve prosthesis according to the preamble of claim 1, which is known e.g. from US-B-6 425 916.

In general, in the fabrication of cardiac valves, and in particular in the fabrication of percutaneous cardiac valves that are to be implanted in the aortic position, it is necessary to reconcile various needs, which are in effect in contrast with one another.

In the first place, it is important to ensure firm anchoring of the valve in the implantation position. In particular, once implanted, the valve must not run the risk of being displaced (dislodged) with respect to the implantation position as a result of the hydraulic thrust exerted by the blood flow.

In the second place, it is important to ensure the tightness of the artificial duct obtained with the valve with respect to the flow duct generically defined by the natural annulus and by the root of the aorta, preventing undesirable ports for flow of the blood from possibly being set up on the outside of the structure of the valve.

Again, as in the case of all cardiac-valve prostheses, it is desirable to have available a structure of prosthetic valve leaflets that can reproduce as faithfully as possible the operation of natural valve leaflets, this as regards minimization of the loss of head induced on the blood flow and as regards the possibility of preventing lines of flow of the blood through the prosthesis from being set up that might depart, as regards their pattern, from the pattern of the lines of flow characteristic of natural blood flow.

Furthermore, it should be taken into account that the methods of implantation of valves via a percutaneous route or by means of thoracic microsurgery are very frequently irrespective of the effective removal of the natural valve leaflets. Very often it is in fact simply envisaged that the cardiac valve is in some way introduced in a position corresponding to the natural annulus and deployed *in situ* by simply divaricating definitively the natural valve leaflets, the functionality of which is destined to be replaced by the valve leaflets of the prosthetic valve.

The purpose of the present invention is to provide a cardiac valve capable of meeting in an excellent way the requirements indicated previously, with particular reference to the possible implantation via percutaneous route or by means of thoracic microsurgery.

According to the present invention, that purpose is achieved thanks to a cardiac-valve prosthesis having the characteristics referred to specifically in claim 1. Advantageous developments of the invention form the subject matter of the subclaims.

In a possible exemplary embodiment, the solution described herein is based on the concept of separating, in the framework of the supporting armature of the valve, the function of anchoring of the valve in the implantation site (including the possible function of sealing the valve with respect to the blood-flow duct natural to the region in which the valve is implanted) by the valve function proper. In a preferred way, that purpose is achieved by providing, within the armature of the valve, two structures (which are functionally distinct, but which in effect may be structurally integrated with one another), which are delegated separately to the accomplishment of these two functions. Envisaged herein is the fact that these two structures or portions comprise an external portion which can be spread out to enable anchorage of the cardiac-valve prosthesis in the implantation site, and an internal portion which is substantially axially coextensive with the external portion for supporting the prosthetic valve leaflets.

The valve thus obtained is adapted in a particularly advantageous way to be implanted in a position corresponding to the so-called Valsalva's sinuses.

The invention will now be described, purely by way of non-limiting example, with reference to the annexed drawings, in which:
- Figure 1 is a general perspective view of the armature of a valve according to the invention;
- Figure 2 illustrates the corresponding valve without the leaflets; and
- Figures 3 and 4 illustrate a second example of embodiment of a valve according to the invention.

In the figures of the annexed sheets of drawings, the reference number 1 designates as a whole a so-called cardiac valve, which can be implanted via percutaneous route or resorting to thoracic-microsurgery techniques.

For a circumstantiated description of the meaning attributed to these terms and of the solutions of valves already proposed in the past, the reader is referred to the introductory part of the present description.

The foregoing also applies as regards the general illustration of the potential advantages linked to the use of cardiac-valve prostheses of this nature and of the corresponding modalities of implantation. It is consequently superfluous herein to proceed to a new detailed exposition of these concepts, which are to be considered certainly known.

The same applies also to a fair extent, with the exception of the elements that will be referred to specifically in what follows, as regards the details of embodiment of a strictly technological nature (choice and treatment of the materials, etc.).

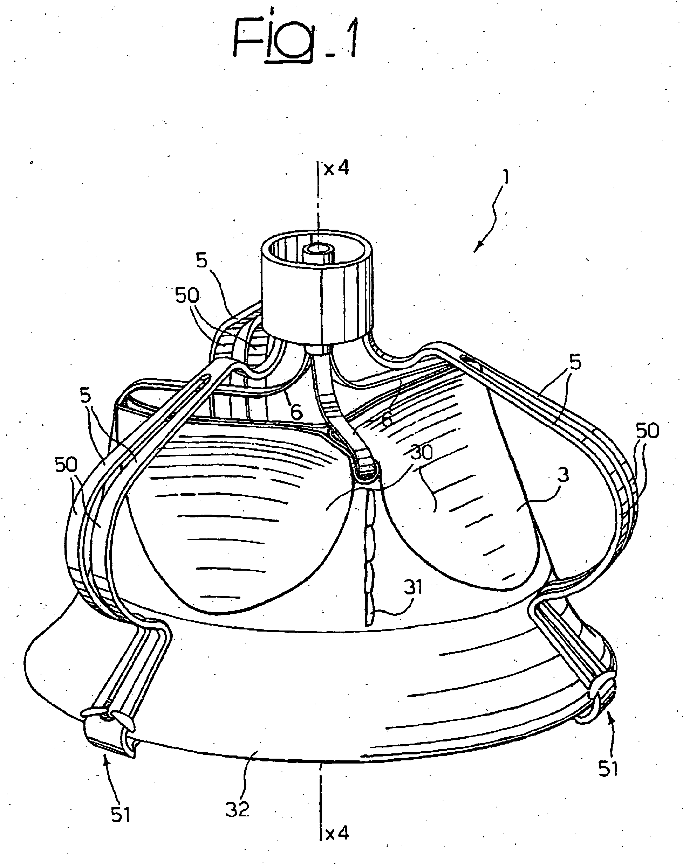

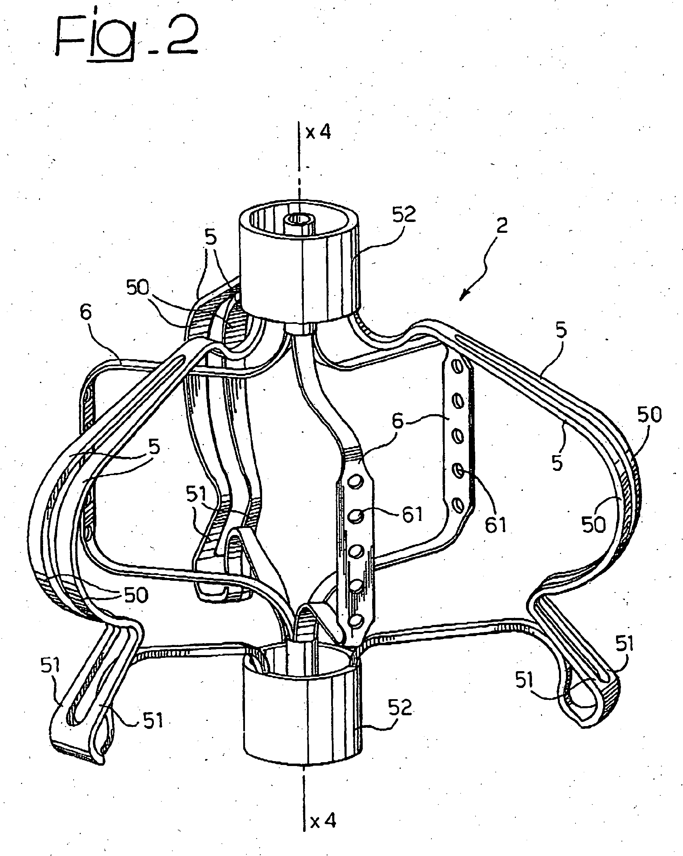

Essentially, the valve 1 represented in Figures 1 and 2 is basically made up of two elements, namely:
- an armature 2, having the characteristics that emerge more clearly from the representation of Figure 2; and
- a set of leaflets 3, coupled to the armature 2 and the characteristics of which will be illustrated in detail in what follows and may be appreciated more fully from the view of Figure 1.

As shown in Figure 2, the armature 2 of the valve has a general cage-like structure comprising a set of ribs that extend with a general symmetry of a cylindrical type about a principal axis X4.

In percutaneous valves, the axis X4 usually corresponds with the principal axis of the distal part of the catheter used for implantation of the valve 1.

For the present purposes, the axis X4 can be viewed basically as an entity of a purely geometrical nature, even though the same can in effect be identified either with elements of the implantation catheter or with elements of the armature 2 of the valve 1.

Basically, within the armature 2 there are distinguishable a first and a second series of ribs, designated, respectively, by the reference numbers 5 and 6.

These ribs are usually made of metal material that is able to present characteristics of radial expandability, both as a result of a positive action of dilatation or divarication exerted via, for example, a balloon catheter and as a result of characteristics proper to the material constituting the ribs 5 (and, possibly, 6), and in particular thanks to the possibility of it being made of a superelastic material and/or a material with shape-memory characteristics. A material that is well known in biomedical applications and that presents the characteristics just cited is the material known as Nitinol.

The possibility of obtaining structures that may substantially be likened to cages, which can be introduced into a vessel of the human body and in particular into a venous site and can then be dilated *in loco* via dilatation of the distal part of the catheter for introduction i.e., (in the case of materials with shape-memory characteristics or the like) as a result of the retraction of a sheath that previously maintains the structure in a compressed position, is well known in the art, having been amply experimented, for example, in stent technology, and in particular the technology of stents for angioplasty.

Concerning the possibility of providing cage-like structures with ribs extending with a profile more or less shaped in diametral planes with reference to a principal axis, such as for example the axis X4, further reference may be made to technologies adopted for the fabrication of devices for the mapping or passivation of endocardial sites, such as, typically, endocavitary sites, used for localization and/or treatment of the so-called ectopic sites characteristic of the pathological conditions of a fibrillative type.

The distinction made - at the level of reference numbers - between the ribs 5 and 6 introduced previously is aimed at taking into account the fact that, within the armature 2, these different sets of ribs are designed to perform different functions, this being true even though these ribs may constitute so many physically distinct parts as different portions of a unitary structure within the armature 2.

In particular, the ribs 5 form an external part or portion of the armature 2 designed (note the representation of Figure 1) to enable the location and anchoring of the valve 1 in the implantation site.

Instead, the ribs 6 are designed to constitute a more internal part of the armature 2, specifically the part that is to support - as will emerge more clearly from what follows - the valve leaflets 30 of the prosthesis.

Basically, the ribs 5 and 6 are designed to define, within the armature 2:
- an external portion (ribs 5), which can be spread out to enable anchorage of the prosthesis 1 in the implantation site; and
- an internal portion (ribs 6), which supports the prosthetic valve leaflets 30 provided in the set of leaflets 3.

An important characteristic of the solution described herein is provided by the fact that the two parts or portions of armature previously described are "substantially axially coextensive" with respect to one another.

This definition is understood to indicate synthetically the fact that these two portions occupy, if observed in their development in the direction of the axis X4, axial stretches that are substantially coincident with one another.

The above is in direct contrast with solutions such as the one described, for example, in US-B-6 482 228: illustrated therein is a valve comprising two homologous portions that are totally staggered with respect to one another in an axial direction so that they can be located, one in a proximal position and the other in a distal position with respect to the coronary ostia.

The ribs 5, which in what follows are designated also as "external" ribs, are preferably arranged in sets of ribs, these sets being typically arranged in threes or multiples of three so as to be more readily adaptable - in a complementary way - to the anatomy of the Valsalva's sinuses, which is the site of choice for implantation of the valve described.

In the exemplary embodiment illustrated herein (which, it is recalled, is merely one example), within the ribs 5 there is distinguishable a top part or top branch 50 and a bottom part or bottom branch 51.

In the implantation position, the top parts or branches 50 are able, when they are in their deployed or expanded position, to extend within the so-called Valsalva's sinuses, i.e., the dilatations, from the overall lobed profile, which are present at the root of the aorta (or the pulmonary valve), hence in a physiologically distal position with respect to the valve annulus.

The bottom portions or branches 51 (note once again the representation of Figure 1) are instead designed to extend in a proximal position with respect to the valve annulus so as to extend for a certain stretch within the ventricular chamber.

As a whole, the top parts 50 of the ribs 5, when brought into the extended position, jointly define a tripodal structure, which, by spreading out within the Valsalva's sinuses, enables attainment of a condition of firm anchorage of the valve 1. This avoids the possibility of the valve 1, once implanted, being dislodged, or even just displaced with respect to the implantation position as a result of the hydraulic stresses applied thereto during operation by the blood flow.

The condition of firm anchorage of the valve 1 in the implantation site is further reinforced by the fact that the area of radiusing between the top branches 50 and the bottom branches 51 of the ribs 5 has, precisely on account of the general divaricated or flared pattern of the bottom branches 51, an area of convexity or loop that is thus positioned astride of the valve annulus, reinforcing the condition of shape fit between the armature of the valve and the implantation site.

It will be appreciated that, in the schematic representation of the figures, the natural valve leaflets of the valve that is to be replaced with the prosthetic valve 1 are not specifically illustrated.

This is intended to highlight the fact that the valve 1 described herein can be located in situ whether the natural valve leaflets have previously been removed or (and in a way that is from certain points of view preferred) without proceeding to the removal of the natural valve leaflets by simply introducing the valve within the valve orifice and thus bringing about, as a result of the divarication of the valve 1 structure, the corresponding definitive divarication of the natural valve leaflets that are brought into - and maintained in - a position of substantial adherence to the surrounding portion of the valve annulus.

It will moreover be appreciated that the particular conformation of the ribs 5, and in particular of the parts 50 and 51 just described, corresponds to the solution envisaged for the implantation of the valve in the aortic site. Structures that are as a whole similar can be adopted for implantations, for example, in the mitral site.

The top and bottom branches 50 and 51 may in actual fact present conformations that are altogether different from the ones illustrated. For example, the top branches 50 may possibly present a conformation that is approximately symmetrical to the conformation represented herein with reference to the bottom branches 51, thus conferring on the external part of the armature 2 of the valve an overall hourglass conformation. It will moreover be appreciated that the presence of both of the branches 50 and 51 is altogether optional.

Again, in the exemplary embodiment of the invention illustrated herein the terminal top ends of the branches 50 and the bottom ends of the branches 51 are connected to collar parts 52, which are designed to be fitted around, and usually to slide along the principal axis X4 during divarication of the armature 2. This solution proves advantageous as regards the simplicity of embodiment and the structural congruence of the armature 2. Recourse thereto should, however, be reconciled with the need to prevent the collar parts 52 from possibly ending up playing an excessive role of obstruction in regard to the blood flow that is to pass (from below upwards, with reference to the configuration of implantation as represented in Figure 1) through the central orifice of the valve defined between the valve leaflets 30 in a divaricated position.

The structure and the configuration of the ribs 6 is, as a whole, akin to that of the ribs 5.

In the case of the ribs 6, which form the internal part of the armature 2 of the valve, there is, however, usually the presence of just three elements that are to support in a position corresponding to homologous lines of commissure (which take on material form as sutures 31 passing through openings 61 provided on the elements 6) of the valve leaflets 30.

Essentially, the complex of ribs 6 and valve leaflets 30 is designed to form the normal structure of a so-called valve prosthesis of a biological type. This is a valve prosthesis which (in the form that is to be implanted with a surgical operation of a traditional type, hence of an invasive nature) has met with a wide popularity in the art.

The structural details of such a valve, consisting of a tubular structure made of biological material (for example, the pericardium or meningeal tissue of animal origin) subjected to treatments of passivation (with gluteraldehyde or similar compounds) or alternatively a biocompatible synthetic material, after prior possible shaping of the cusp-like areas that are to constitute the prosthetic valve leaflets 30, are amply known in the art.

To illustrate the detailed structure of such a valve, useful reference can be made to EP-B-0 155 245, whilst as regards the technology of fabrication and/or treatment of the material of the prosthetic valve leaflets, useful reference can be made to EP-B-0 133 420.

In addition to the more strictly "valve" part comprising the prosthetic valve leaflets 30 supported in a commissural position by the ribs 6 of the armature 2, the leaflet part of the prosthesis illustrated also has an apron-like part 32, which extends according to a general chimney-like or flared configuration, being supported by the bottom parts 51 of the external ribs 5 of the armature 2.

For this purpose, the bottom parts 51 have an overall V-shaped structure, hence comprising two branches that are to enclose within them the chimney-like portion 32 of the set of leaflets of the valve armature 2, so causing this to be divaricated at the moment of spreading-out of the valve and maintained in such divaricated position in the intraventricular site in a proximal position with respect to the valve annulus.

Albeit conserving an extreme structural simplicity, from which there derives a corresponding reliability during implantation, the valve structure described manages to meet in an excellent way various needs that in themselves contrast with one another.

This is obtained basically by separating the two parts of the armature (which are functionally distinct, even though they may be integrated in a single structure) and hence delegating to them two different functions.

In particular, the external ribs 5 (and more specifically when these are present in the two branches 50 and 51, which can set themselves astride of the valve annulus) provide the firm anchoring of the valve *in situ,* so preventing it from possibly being removed or even just displaced from the implantation site chosen by the person carrying out the implantation operation.

This is obtained with a structure that is able to adapt to the natural anatomical conformation without exerting thereon stresses of a traumatic nature.

As may be appreciated from the attached sheets of drawings, the ribs 5 can present a rather thin or light structure. This causes them possibly to adhere to the walls of the root of the aorta, to the surrounding portion of the valve annulus, and to the walls of the endocardial chamber, without exerting particularly marked stresses on those walls, which - in extreme cases - could even be at the basis of phenomena of lesions and, even more, of onset of reactions of the organism in regard to these lesions. The action of anchoring is in fact achieved, more than for any other reason, on account of the presence of numerous ribs 5 and their conformation, which is complementary with respect to that of the implantation site (in particular, when this is represented by the Valsalva's sinuses).

The internal part of the armature 2, represented by the ribs 6, enables, instead, spreading-out and support of the prosthetic valve leaflets 30 in the implantation position. This is achieved by recourse to a structure and a conformation which, precisely because they reproduce very closely those of traditional prosthetic valves of a biological type, prove particularly suitable and efficient for performing substitution of a defective natural valve. In particular, it is well known the fact that valve prostheses of a biological type, precisely because they are such as to reproduce much more closely the fluid-dynamic characteristics and the characteristics of behaviour of the leaflets of natural cardiac valves, can be used to benefit also in patients affected by forms of rather marked cardiac insufficiency.

It will be appreciated that this possibility is achieved in an optimal way in the solution described herein precisely because the valve function is rendered altogether independent of the function of anchoring in situ of the prosthesis.

The presence of the branches 51 in the armature 2 of the prosthesis and, conversely, of the apron-like or chimney-like portion 32 in the set of leaflets 3 (in addition to contributing further to the shape fit and hence to the anchoring in the implantation site of the valve 1) likewise enables very efficient channelling of the blood flow coming from the heart, channelling it in a practically complete way, precisely on account of the divaricated or flared configuration of the chimney-like portion 32 within the flow duct defined within the valve leaflets 30. There is thus minimized (and in effect cancelled out) in times shortly following implantation, the possibility of there being created lines of blood flow that are directed from the cardiac chamber towards the vessel that exits therefrom and are such as to pass on the outside of the cardiac-valve prosthesis.

The foregoing likewise envisages that the two parts or portions of armature previously described are substantially axially coextensive with respect to one another, occupying, if viewed in their development in the direction of the axis X4, axial stretches substantially coinciding with one another. This characteristic enables a precise positioning of the prosthetic valve leaflets in a "physiological" location (i.e., in a location basically corresponding to the location of the natural valve leaflets), likewise benefiting, for the purposes of the anchoring of the prosthesis in situ, from a shape fit with the Valsalva's sinuses.

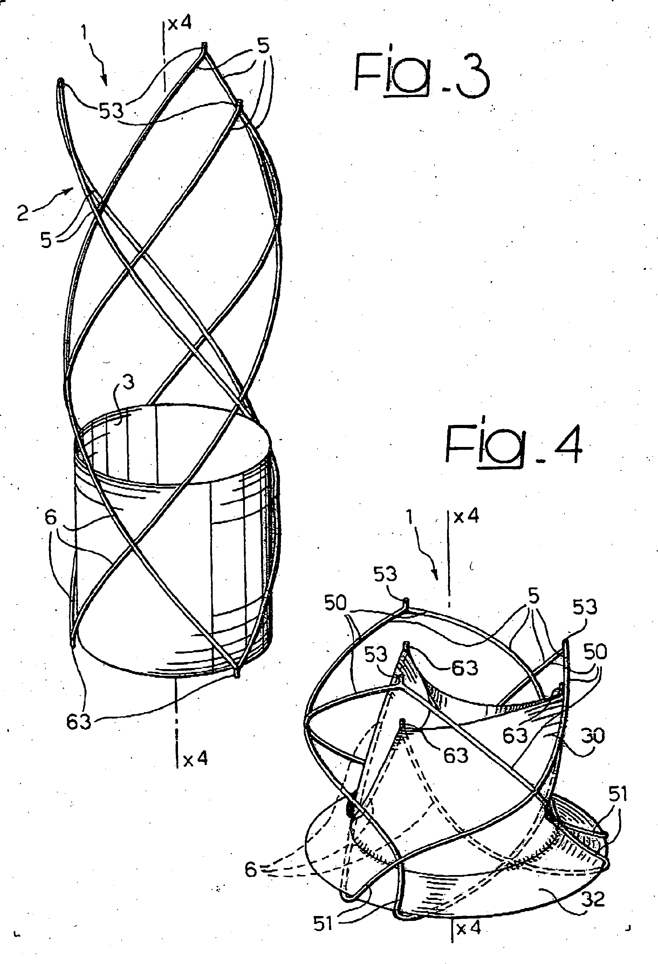

Figures 3 and 4 illustrate a second exemplary embodiment of a cardiac valve of the type described herein.

From the general structural standpoint, the valve of Figures 3 and 4 stems basically from the structure of the valve described previously with reference to Figures 1 and 2.

The valve according to Figures 3 and 4 is to be obtained by recourse, as regards the armature, to a single element of tubular shape and reticular structure.

In particular, Figure 3 illustrates this tubular element in an "extroverted" or "extended" configuration, whereas in Figure 4 the same element is illustrated in an "introverted" configuration, corresponding to the final conformation of implantation of the valve.

In this particular embodiment, the two parts or portions of the armature 2 (the external portion 5 for anchoring of the valve 1 in the implantation site and the internal portion 6 with the function of supporting the valve leaflets) are integrated in a single structure consisting precisely of the tubular element referred to previously.

That element, designated as a whole by 2, has a substantially cylindrical structure that develops about a principal axis X4 and consists of ribs having a general helical conformation and presenting joints in pairs 53 and 63 at the two ends. The armature 2 is without any discontinuity between the external portion 5 and the internal portion 6 of the ribs.

The ribs that form the element 2 in this example of embodiment are basically made of a superelastic material and/or shape-memory material.

The valve 1 passes from the extroverted configuration (Figure 3) to the introverted or final configuration (Figure 4) through a mechanism of deformation that may be obtained at the implantation site. This can occur, in the case of percutaneous valves, via remote manipulation devices associated to the catheter for introduction of the valve. Such devices are in themselves known in so far as they are used in association with catheters of various types, for example to obtain actions of selective spreading-out of implantation devices, such as stents, stent-grafts or the like.

The mechanism of deployment (i.e. "introversion") in question envisages passing from the extended conformation of Figure 3 to the final conformation of Figure 4, thereby causing the joints 63 - to which the set of leaflets 3 are fixed (internally with respect to the original cylindrical tubular element) - to converge within the tubular structure and then advance therein until the joints 63 arrive in the proximity of the joints 53, which, at the start of the movement of introversion described, were exactly at the opposite end of the original cylindrical tubular element.

Basically, this movement causes the tubular element 2 to pass between:
- an extroverted configuration (Figure 3), in which the tubular element has a substantially cylindrical shape, with the external portion 5 and internal portion 6 of the armature axially juxtaposed with respect to one another, and
- a final introverted configuration (Figure 4), in which the aforesaid external portion 5 and internal portion 6 are substantially axially coextensive with respect to one another.

This overall movement is usually accompanied by at least a slight divarication of the joints 53 and of the areas of the armature of the valve (external part 5) adjacent thereto.

That divarication of the joints 53 and of the areas of the armature adjacent thereto is clearly perceptible in Figure 4.

In the same figure there is moreover perceptible the action of "pinching" of the apron-like part of the leaflet, which may be achieved with a positive action of deformation of the armature and/or by exploiting the shape-memory characteristics of the constituent material.

It will be noted that, also in this case, a final configuration is reached, in which the external portion 5 and internal portion 6 of the armature of the valve 1 are substantially axially coextensive with respect to one another, finding themselves occupying axial stretches that substantially coincide with respect to the axis X4.

The introverted configuration of Figure 4 has the ribs 5, in which it is possible to distinguish top branches 50 and bottom branches 51, in a conformation suitable for being implanted in the Valsalva's sinuses. The top branches 50 extend in a generally cage-like form, which is complementary with the lobed anatomy of the Valsalva's sinuses and co-operate with the bottom branches 51, which are to extend in a proximal position with respect to the valve annulus so as to be introduced within the ventricular chamber, ensuring a firm anchoring of the valve 1 at the implantation site.

The set of leaflets 3 comprises the prosthetic valve leaflets 30 supported by the internal ribs 6 and an apron-like part 32 supported by/fixed to the bottom branches 51 of the external ribs 5.

Of course, without prejudice to the principle of the invention, the details of construction and the forms of embodiment may vary widely with respect to what is described and illustrated herein, without thereby departing from the scope of the present invention, as defined by the annexed claims.

## Claims

1. A cardiac-valve prosthesis, comprising:
- an armature (2) adapted for deployment in an expanded implantation position, and
- a set of leaflets (3) coupled to said armature (2) for deployment therewith in said expanded position; said set of leaflets (3) being able to define, in said expanded position, a flow duct for the blood selectively obstructable by said set of prosthetic valve leaflets (30) as a result of the reversal of the direction of flow of said blood flow,
wherein said armature (2) comprises:
- an external portion (5), adapted for deployment to enable the anchorage of the cardiac-valve prosthesis at the implantation site; and
- an internal portion (6), which supports said prosthetic valve leaflets (30) ; said external portion (5) and said internal portion (6) being substantially axially coextensive with respect to one another.
**characterized in that** said external portion (5) of said armature (2) has a set of ribs (5) which, in said expanded position, are able to cooperate in a shape fit relationships, with the implantation site, wherein said ribs (5) in said external portion of said armature have terminal ends connected to coller parts (52).

2. The prosthesis according to Claim 1, **characterized in that** said external portion (5) and said internal portion (6) of said armature (2) are structurally distinct from one another.

3. The prosthesis according to Claim 1, **characterized in that** said external portion (5) and said internal portion (6) of said armature (2) form different parts of a single structure.

4. The prosthesis according to any one of the preceding Claims 1 to 3, **characterized in that** said armature (2) has an overall cage-like structure.

5. The prosthesis according to Claim 1, **characterized in that** said ribs (5) have at least one portion (50) having an overall arched pattern.

6. The prosthesis according to either Claim 1 or Claim 5, **characterized in that** said ribs (5), of said external portion (5) of the armature have branches (51) that are able to extend in an endocardial position.

7. The prosthesis according to any one of Claims 1, 5 or 6, **characterized in that** said ribs (5) of said external portion (5) of the armature (2) have parts that are able to extend astride of a valve annulus site.

8. The prosthesis according to any one of Claims 1, 5, 6, or 7 to 8, **characterized in that** said ribs (5) of said external portion (5) of said armature (2) comprise, respectively, a first branch (50) and a second branch (51) that are able to extend, respectively, in a distal position and a proximal position with respect to a valve annulus site.

9. The prosthesis according to any one of Claims 1, 5, 6, 7, or 8 **characterized in that** said ribs (5) of said external portion (5), of the armature are grouped together into sets, said sets being in threes or multiples of three.

10. The prosthesis according to any one of the preceding claims, **characterized in that** said internal portion (6) of said armature (2) comprises respective ribs (6) that are able to support said prosthetic valve leaflets (3) in a commissural position.

11. The prosthesis according to any one of the preceding claims, **characterized in that** said internal portion (6) of said armature (2) comprises ribs provided with openings (61) for the passage of formations (31) for anchoring of said prosthetic valve leaflets (3).

12. The prosthesis according to any one of the preceding claims, **characterized in that** said internal portion (6) of said armature (2) comprises ribs (6) grouped together into sets, said sets being in threes or multiples of three.

## Patentansprüche

1. Eine Herzklappenprothese, die umfasst:
- einen Rahmen (2), der für einen Einsatz in einer expandierten Implantationsposition geeignet ist, und
- einen Satz Blättchen (3), der mit dem Rahmen (2) gekoppelt ist, um mit diesem in besagter expandierter Position eingesetzt zu werden; wobei besagter Satz Blättchen (3) in der Lage ist, in besagter expandierter Position einen Strömungskanal für das Blut zu definieren, der durch besagten Satz prothetischer Ventilblättchen (30) als ein Ergebnis der Umkehr der Strömungsrichtung von besagtem Blutstrom selektiv versperrbar ist,
wobei besagter Rahmen (2) umfasst:
- einen externen Bereich (5), der für den Einsatz angepasst ist, um die Verankerung der Herzklappenprothese an dem Implantationsort zu ermöglichen; und
- einen internen Bereich (6), der besagte prothetische Ventilblättchen (30) hält, wobei der externe Bereich (5) und der interne Bereich (6) im wesentlichen axial koextensiv bezogen aufeinander sind,
**dadurch gekennzeichnet, dass** der externe Bereich (5) des Rahmens (2) einen Satz Rippen (5) aufweist, die in der expandierten Position in der Lage sind, in einer formschlüssigen Beziehung mit dem Implantationsort zusammenzuwirken, wobei die Rippen (5) in dem externen Bereich des Rahmens terminale Enden aufweisen, die mit Kragenteilen (52) verbunden sind.

2. Die Prothese nach Anspruch 1, **dadurch gekennzeichnet, dass** der externe Bereich (5) und der interne Bereich (6) des Rahmens (2) strukturell voneinander getrennt sind.

3. Die Prothese nach Anspruch 1, **dadurch gekennzeichnet, dass** der externe Bereich (5) und der interne Bereich (6) des Rahmens (2) verschiedene Teile einer einzelnen Struktur bilden.

4. Die Prothese nach einem der vorhergehenden Ansprüche 1 bis 3, **dadurch gekennzeichnet, dass** der Rahmens(2) eine insgesamt käfigartige Struktur aufweist.

5. Die Prothese nach Anspruch 1, **dadurch gekennzeichnet, dass** die Rippen (5) wenigstens einen Bereich (50) aufweisen, der ein insgesamt bogenförmiges Muster aufweist.

6. Die Prothese nach Anspruch 1 oder Anspruch 5, **dadurch gekennzeichnet, dass** die Rippen (5) des externen Bereichs (5) des Rahmens Zweige (51) aufweisen, die in der Lage sind, sich in einer endokardialen Position zu erstrecken.

7. Die Prothese nach einem der Ansprüche 1, 5 oder 6, **dadurch gekennzeichnet, dass** die Rippen (5) des externen Bereichs (5) des Rahmens (2) Teile aufweisen, die in der Lage sind, sich rittlings von einer Ventilkranzseite zu erstrecken.

8. Die Prothese nach einem der Ansprüche 1, 5, 6 oder 7, **dadurch gekennzeichnet, dass** die Rippen (5) des externen Bereichs (5) des Rahmens (2) jeweils einen ersten Zweig (50) bzw. einen zweiten Zweig (51) umfassen, die in der Lage sind, sich bezogen auf eine Ventilkranzseite entsprechend in einer distalen Position und einer proximalen Position zu erstrecken.

9. Die Prothese nach einem der Ansprüche 1, 5, 6, 7 oder 8, **dadurch gekennzeichnet, dass** die Rippen (5) des externen Bereichs (5) des Rahmens in Sätzen zusammen gruppiert sind, wobei besagte Sätze dreier oder vielfache von drei sind.

10. Die Prothese nach einem der vorhergehenden Ansprüche, **dadurch gekennzeichnet, dass** der interne Bereich (6) des Rahmens (2) entsprechende Rippen (6) umfasst, die in der Lage sind, besagte prothetische Ventilblättchen (3) in einer kommisuralen Position zu halten.

11. Die Prothese nach einem der vorhergehenden Ansprüche, **dadurch gekennzeichnet, dass** der interne Bereich (6) des Rahmens (2) Rippen umfasst, die mit Öffnungen (61) für den Durchgang von Formationen (31) für die Verankerung von besagten prothetischen Ventilblättchen (3) ausgeführt sind.

12. Die Prothese nach einem der vorhergehenden Ansprüche, **dadurch gekennzeichnet, dass** der interne Bereich (6) des Rahmens (2) Rippen (6) umfasst, die in Sätzen zusammen gruppiert sind, wobei besagte Sätze dreier oder vielfache von drei sind.

## Revendications

1. Prothèse de valvule cardiaque, comprenant :
- une armature (2) adaptée au déploiement dans une position d'implantation étendue, et
- un ensemble de feuillets (3) reliés à ladite armature (2) pour un déploiement avec celle-ci dans ladite position étendue ; ledit ensemble de feuillets (3) pouvant délimiter, dans ladite position étendue, un conduit d'écoulement pour le sang, pouvant être sélectivement obstrué par ledit ensemble de feuillets de valvule prothétique (30) par suite de l'inversion de la direction d'écoulement du dit courant sanguin,
dans laquelle ladite armature (2) comprend :
- une partie externe (5), adaptée à être déployée pour permettre l'ancrage de la prothèse de valvule cardiaque au site d'implantation ; et
- une partie interne (6) qui supporte lesdits feuillets de valvule prothétique (30) ; ladite partie externe (5) et ladite partie interne (6) s'étendant à peu près coaxialement l'une par rapport à l'autre,
**caractérisée en ce que** ladite partie externe (5) de ladite armature (2) comporte un ensemble de bras (5), qui, dans ladite position étendue, peuvent coopérer par adaptation de forme avec le site d'implantation, dans laquelle lesdits bras (5) dans ladite partie externe de ladite armature ont des extrémités reliées à des parties de manchon (52).

2. Prothèse selon la revendication 1, **caractérisée en ce que** ladite partie externe (5) et ladite partie interne (6) de ladite armature (2) sont structuralement distinctes l'une de l'autre.

3. Prothèse selon la revendication 1, **caractérisée en ce que** ladite partie externe (5) et ladite partie interne (6) de ladite armature (2) forment des parties différentes d'une unique structure.

4. Prothèse selon l'une quelconque des revendications 1 à 3, **caractérisée en ce que** ladite armature (2) a une structure générale en cage.

5. Prothèse selon la revendication 1, **caractérisée en ce que** lesdits bras (5) comportent au moins une partie (50) ayant une forme générale cintrée.

6. Prothèse selon la revendication 1 ou la revendication 5, **caractérisée en ce que** lesdits bras (5) de ladite partie externe (5) de l'armature comportent des branches (51) qui peuvent s'étendre dans une position endocardiaque.

7. Prothèse selon l'une quelconque des revendications 1, 5 ou 6, **caractérisée en ce que** lesdits bras (5) de ladite partie externe (5) de l'armature (2) ont des parties qui peuvent s'étendre en travers d'un site d'annulus valvulaire.

8. Prothèse selon l'une quelconque des revendications 1, 5, 6 ou 7, **caractérisée en ce que** lesdits bras (5) de ladite partie externe (5) de ladite armature (2), comprennent respectivement une première branche (50) et une deuxième branche (51) qui peuvent respectivement s'étendre dans une position distale et une position proximale par rapport à un site d'annulus valvulaire.

9. Prothèse selon l'une quelconque des revendications 1, 5, 6, 7 ou 8, **caractérisée en ce que** lesdits bras (5) de ladite partie externe (5) de l'armature sont rassemblés en ensembles lesdits ensembles étant de trois ou de multiples de trois.

10. Prothèse selon l'une quelconque des revendications précédentes, **caractérisée en ce que** ladite partie interne (6) de ladite armature (2) comprend des bras respectifs (6) qui peuvent supporter lesdits feuillets de valvule prothétique (3) dans une position commissurale.

11. Prothèse selon l'une quelconque des revendications précédentes, **caractérisée en ce que** ladite partie interne (6) de ladite armature (2), comprend des bras pourvus d'ouvertures (61) pour le passage d'organes (31) pour l'ancrage des dits feuillets de valvule prothétique (3).

12. Prothèse selon une quelconque des revendications précédentes, **caractérisée en ce que** ladite partie interne (6) de ladite armature (2) comprend des bras (6) rassemblés en ensembles, lesdits ensembles étant de trois ou de multiples de trois.
